# EUROPÄISCHE PATENTANMELDUNG

(11) **EP 2 926 882 A1**
(43) Veröffentlichungstag der Anmeldung: **07.10.2015**
(21) Anmeldenummer: 14001211.3
(22) Anmeldetag: 01.04.2014
(51) Int. Cl.: B01D 53/00, B01D 3/14, B01D 5/00, C07C 7/04, C07C 7/09, F25J 3/02

(54) **Verfahren und Anlage zur Trennung eines Gasgemischs und Verfahren zum Umrüsten einer Trennanlage**

(71) Anmelder: Linde Aktiengesellschaft, 80331 München (DE)
(72) Erfinder: Fritz, Helmut, 81375 München (DE); McCracken, Sean, 82178 Puchheim (DE); Boese, Christoph, 80333 München (DE); Bezold, Theresia, 81377 München (DE)

(57) **Zusammenfassung**

Ein Verfahren zur Trennung eines Gasgemischs, das überwiegend Wasserstoff, Methan, Ethan und Ethylen und wahlweise weitere Kohlenwasserstoffe enthält, unter Verwendung einer Destillationssäule (122) wird vorgeschlagen, bei dem das Gasgemisch von einem ersten Temperaturniveau T1 auf ein zweites Temperaturniveau T2, das zwischen den Kondensationstemperaturen von Ethylen und Methan liegt, abgekühlt wird, wobei zumindest ein Kondensat aus dem Gasgemisch abgeschieden wird, aus dem zumindest einen Kondensat zumindest ein überwiegend Methan, Ethan und/oder Ethylen enthaltender Strom (t, d, f, h) gebildet und zumindest teilweise in die Destillationssäule (122) eingespeist wird, in der Destillationssäule (122) ein überwiegend Methan und Wasserstoff und ferner Ethylen enthaltendes Kopfprodukt erzeugt und zumindest teilweise als Kopfstrom (u) aus der Destillationssäule (122) abgezogen wird, und aus dem Kopfstrom (u) nach weiterem Abkühlen zumindest eine ethylenangereicherte Flüssigkeit abgeschieden und zumindest teilweise als Rücklaufstrom (v) auf die Destillationssäule (122) aufgegeben wird. Es ist vorgesehen, dass für das weitere Abkühlen des Kopfstroms (u) zumindest ein Teilstrom (I) eines auf dem zweiten Temperaturniveau T2 gasförmig bleibenden Anteils des Gasgemischs verwendet wird, wobei der der Teilstrom (I) anschließend zumindest teilweise entspannt wird. Der Mengenstrom des zum Abkühlen des Kopfstroms (u) verwendeten Teilstroms (I) kann in Abhängigkeit von einem Wasserstoffgehalt, einem Methangehalt und/oder einem Methan-Wasserstoff-Verhältnis des Gasgemischs eingestellt werden, um das zweite Temperaturniveau T2 zu minimieren. Eine entsprechende Trennanlage (100) und ein Verfahren zum Umrüsten einer Trennanlage sind ebenfalls Gegenstand der Erfindung.

## Beschreibung

Die Erfindung betrifft ein Verfahren zur Trennung eines Gasgemischs, das überwiegend Wasserstoff, Methan, Ethan und Ethylen und wahlweise weitere Kohlenwasserstoffe enthält, eine Trennanlage und ein Verfahren zum Umrüsten einer Trennanlage gemäß den Oberbegriffen der unabhängigen Patentansprüche.

### Stand der Technik

Verfahren und Vorrichtungen zum Dampfspalten (engl. Steam Cracking) von Kohlenwasserstoffen sind bekannt und beispielsweise im Artikel "Ethylene" in Ullmann's Encyclopedia of Industrial Chemistry, online seit 15. April 2007, DOI 10.1002/14356007.a10_045.pub2, beschrieben.

Dampfspaltverfahren werden im kommerziellen Maßstab in Rohrreaktoren durchgeführt, die grundsätzlich mit einer Vielzahl von Kohlenwasserstoffen und Kohlenwasserstoffgemischen von Ethan bis Gasöl bis zu einem Siedepunkt von typischerweise 600 °C (sogenannter Ofeneinsatz) beschickt werden können. Unter gleichen oder vergleichbaren Spaltbedingungen (siehe hierzu unten) betriebene Reaktionsrohre oder Gruppen von Reaktionsrohren, gegebenenfalls aber auch unter gleichen oder vergleichbaren Spaltbedingungen betriebene Rohrreaktoren insgesamt, werden nachfolgend jeweils als "Spaltöfen" bezeichnet. Ein Spaltofen ist im hier verwendeten Sprachgebrauch also eine zum Dampfspalten verwendete bauliche Einheit, die einen Ofeneinsatz gleichen oder vergleichbaren Spaltbedingungen aussetzt. Eine Anlage zum Dampfspalten kann einen oder mehrere derartiger Spaltöfen aufweisen.

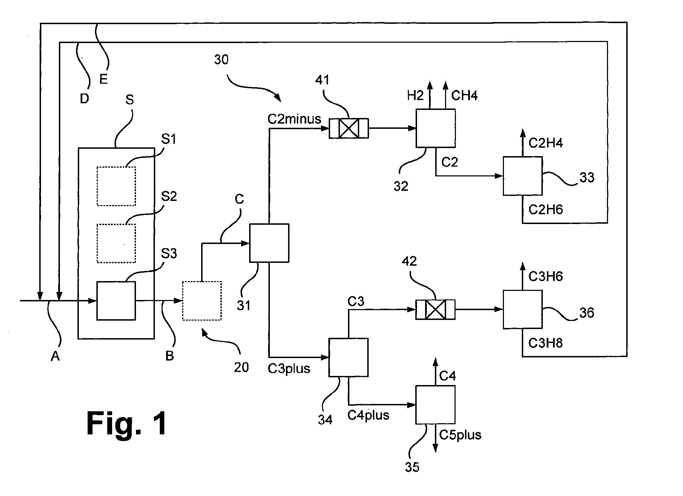

Der jeweilige Ofeneinsatz wird beim Dampfspalten in einem oder mehreren gleich oder unterschiedlich betriebenen Spaltöfen zumindest teilweise umgesetzt, wodurch ein sogenanntes Rohgas erhalten wird. Das Rohgas mehrerer Spaltöfen kann zusammengefasst und, wie auch unter Bezugnahme auf die Figur 1 erläutert, einer Reihe von Nachbehandlungsschritten unterworfen werden. Derartige Nachbehandlungsschritte umfassen zunächst eine Aufbereitung des Rohgases, beispielsweise durch Quenchen, Kühlen und Trocknen, wodurch ein sogenanntes Spaltgas erhalten wird. Bisweilen wird auch bereits das Rohgas als Spaltgas bezeichnet und umgekehrt.

Bei dem Spaltgas handelt es sich um ein Gasgemisch aus Wasserstoff und Kohlenwasserstoffen unterschiedlicher Kohlenstoffanzahl und Struktur. Um aus dem Spaltgas die erwünschten Produkte zu gewinnen, muss dieses daher in Fraktionen aufgetrennt werden. Aus dem Stand der Technik sind hierzu unterschiedliche Verfahren bekannt und beispielsweise in dem erwähnten Artikel "Ethylene" in Ullmann's Encyclopedia of Industrial Chemistry ausführlich beschrieben.

Wie unten ebenfalls erläutert, setzt sich in einer Dampfspaltanlage der Ofeneinsatz eines oder mehrerer Spaltöfen typischerweise aus einem oder mehreren von extern zugeführten sogenannten Frischeinsätzen und aus einem oder mehreren aus dem Spaltgas abgetrennten Recycleströmen zusammen.

Die Zusammensetzung des Spaltgases ergibt sich unter anderem aus der Zusammensetzung des jeweils verwendeten Ofeneinsatzes. Je ethanreicher der oder die im Ofeneinsatz verwendeten Frischeinsätze sind, desto mehr Ethan enthält auch das Spaltgas. Somit finden sich im Spaltgas bei der Verwendung ethanreicher Kohlenwasserstoffgemische deutlich höhere Anteile an Ethan wieder als bei der Verwendung ethanarmer Kohlenwasserstoffgemische wie Naphtha.

Gleichzeitig ergibt sich ein im Vergleich zu herkömmlichen Einsätzen wie Naphtha deutlich erhöhter Wasserstoffgehalt im Spaltgas. Der Gehalt an Methan, sowohl bezogen auf den Wasserstoffgehalt als auch bezogen auf den Gehalt an Ethan und Ethylen, verringert sich hingegen deutlich. Es kommt, mit anderen Worten, selbst bei gleichbleibender Ethylenkapazität zu einer signifikanten Veränderung der Zusammensetzung des Spaltgases. Dies kann insbesondere in bestehenden Anlagen zu Problemen führen, wie auch nachfolgend noch erläutert.

Jedoch kann es aus wirtschaftlichen Gründen wünschenswert sein, ethanreiche Kohlenwasserstoffgemische als Frischeinsätze zu verwenden. Diese fallen bei der Erdgasproduktion in großen Mengen an, unter anderem in Form sogenannter Erdgaskondensate (engl. Natural Gas Liquids, NGL), und können durch Dampfspalten zu Wertprodukten umgesetzt werden. Entsprechendes gilt auch für das durch Frackingverfahren geförderte vergleichsweise ethanreiche Schiefergas (engl. Shale Gas). Insbesondere die Umstellung bestehender Anlagen ist bisweilen gewünscht.

Vor diesem Hintergrund besteht daher der Bedarf nach technischen Möglichkeiten, die eine Umstellung entsprechender Anlagen auf ethanreichere Frischeinsätze erlauben.

### Offenbarung der Erfindung

Vor diesem Hintergrund schlägt die vorliegende Erfindung ein Verfahren zur Trennung eines Gasgemischs, das überwiegend Wasserstoff, Methan, Ethan und Ethylen und wahlweise weitere Kohlenwasserstoffe enthält, eine entsprechende Trennanlage und ein Verfahren zum Umrüsten einer Trennanlage mit den Merkmalen der unabhängigen Patentansprüche vor. Bevorzugte Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen weiter erläutert und die verwendeten Begriffe definiert.

Im Extremfall, also bei der überwiegenden oder ausschließlichen Verwendung von Ethan als Frischeinsatz beim Dampfspalten, kann gegenüber herkömmlichen Frischeinsätzen die zwei- bis vierfache Menge an Wasserstoff im Spaltgas enthalten sein. Gleichzeitig enthält das Spaltgas, wie erwähnt, erheblich weniger Methan als bei der Verwendung von schwereren Frischeinsätzen.

Die Folge der Änderung der absoluten Menge und Konzentration der genannten Komponenten ist, dass bestehende Zerlegungsanlagen für das Spaltgas häufig nicht mehr in der Lage sind, die Ethylenverluste ausreichend zu begrenzen. Ethylen geht in diesem Fall in zu großen Anteilen in eine aus dem Spaltgas gebildete Fraktion über, die ansonsten Methan, gegebenenfalls auch Wasserstoff, enthält.

In bekannten Anlagen zum Dampfspalten wird beispielsweise zunächst eine Fraktion gebildet, die überwiegend Wasserstoff, Methan, Ethan und Ethylen enthält. Höhere Kohlenwasserstoffe werden zuvor abgetrennt.

Aus dieser Fraktion wird Wassserstoff weitgehend abgetrennt, so dass der verbleibende Anteil sich überwiegend aus Methan, Ethan und Ethylen zusammensetzt. Die Auftrennung dieses verbleibenden Anteils beginnt mit der Einspeisung in eine Trennsäule (sogenannte C1/C2-Trennung). Im Sumpf dieser Trennsäule reichern sich Kohlenwasserstoffe mit zwei Kohlenstoffatomen (überwiegend Ethan und Ethylen), am Kopf reichert sich überwiegend Methan an.

Aufgrund der erläuterten Verringerung der Methanmenge (Wasserstoff wurde, wie erwähnt, bereits zuvor weitgehend abgetrennt) befindet sich bei der Umstellung des Frischeinsatzes auf ethanreich(er)e Kohlenwasserstoffe der Kopfbereich der genannten Trennsäule in Unterlast. Hierdurch wird die Kondensation im Kopfkondensator der Trennsäule erschwert. Aus einem im Kopfbereich der Destillationssäule abgezogenen gasförmigen Kopfstrom kann daher nicht ausreichend Ethylen abgeschieden und als Rücklauf auf die Destillationssäule zurückgeführt werden. Das in der Gasphase verbleibende Ethylen geht in die Methanfraktion über und ist damit praktisch verloren.

Daher sind bestehende Anlagenkonfigurationen häufig unter energetischen und trenntechnischen Aspekten suboptimal bis ungeeignet für die Verwendung ethanreich(er)er Frischeinsätze. Stofflich ausgedrückt ist die Methanmenge hier zu gering, um genügend Kälte auf ausreichend tiefem Temperaturniveau zu erzeugen. Die Erfindung schafft hier durch die unten erläuterten Maßnahmen Abhilfe.

Entsprechende Probleme treten selbstverständlich nicht nur dann auf, wenn herkömmliche Frischeinsätze wie beispielsweise Naphtha vollständig durch ethanreich(er)e Frischeinsätze ersetzt werden, sondern auch bei einer nur teilweisen Verwendung ethanreich(er)er Frischeinsätze. Die durch den Einsatz ethanreicher Frischeinsätze erzielbaren wirtschaftlichen Vorteile werden durch die ungeeignete Anlagenkonfiguration wieder zunichte gemacht.

Die vorliegende Erfindung schlägt daher vor, durch zusätzliche Kälte und gezielte Kälteintegration am Kopf der Destillationssäule der fehlenden Methanmenge entgegenzuwirken und den Überschuss an Wasserstoff zumindest teilweise zu entspannen, wodurch zusätzliche Kälte generiert werden kann. Ziel der Erfindung ist es, die Ethylenverluste zu minimieren.

Gängige Verfahren umfassen insbesondere die Trennung des Spaltgases in eine Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (jedoch konventionsgemäß unter Umständen auch Wasserstoff, dann auch "C1minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für die "C5-Fraktion" und die höheren Fraktionen. Mehrere solcher Fraktionen können auch verfahrens- und/oder bezeichnungsmäßig zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

Die vorliegende Anmeldung verwendet zur Charakterisierung von Drücken und Temperaturen die Begriffe "Druckniveau" und "Temperaturniveau", wodurch zum Ausdruck gebracht werden soll, dass Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Flüssige und gasförmige Ströme können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1 %, 0,1 %, 0,01 % oder 0,001 % auf molarer, Gewichts- oder Volumenbasis stehen kann. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält. Ein "überwiegend" eine oder mehrere Komponenten aufweisender Strom enthält diese eine oder mehreren Komponenten beispielsweise im oben mit "reich" definierten Umfang.

Ein flüssiger oder gasförmiger Strom ist von einem anderen flüssigen oder gasförmigen Strom (auch als Ausgangsstrom bezeichnet) "abgeleitet", wenn er zumindest einige in dem Ausgangsstrom enthaltene oder aus diesem erhaltene Komponenten aufweist. Ein in diesem Sinne abgeleiteter Strom kann aus dem Ausgangsstrom durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten werden.

Ein "Wärmetauscher" dient zur indirekten Übertragung von Wärme zwischen zumindest zwei z.B. im Gegenstrom zueinander geführten Strömen, beispielsweise einem zu trennenden Gasgemisch und einem flüssigen C2- oder C3-Strom. Ein Wärmetauscher kann aus einem einzelnen oder mehreren parallel und/oder seriell verbundenen Wärmetauscherabschnitten gebildet sein, z.B. aus einem oder mehreren Plattenwärmetauscherblöcken. Ein Wärmetauscher weist "Passagen" auf, die als voneinander getrennte Fluidkanäle mit Wärmeaustauschflächen ausgebildet sind.

Eine "Entspannungsturbine" bzw. "Entspannungsmaschine", die über eine gemeinsame Welle mit weiteren Entspannungsturbinen oder Energiewandlern wie Ölbremsen, Generatoren oder Verdichtern gekoppelt sein kann, ist zur Entspannung eines gasförmigen oder zumindest teilweise flüssigen Stroms eingerichtet. Insbesondere können Entspannungsturbinen zum Einsatz in der vorliegenden Erfindung als Turboexpander ausgebildet sein. Wird ein Verdichter mit einer oder mehreren Entspannungsturbinen angetrieben, jedoch ohne extern, beispielsweise mittels eines Elektromotors, zugeführte Energie, wird der Begriff "turbinengetriebener" Verdichter oder alternativ "Booster" verwendet. Anordnungen aus turbinengetriebenen Verdichtern und Entspannungsturbinen werden auch als "Boosterturbinen" bezeichnet.

### Vorteile der Erfindung

Die Erfindung geht von einem Verfahren zur Trennung eines Gasgemischs, das überwiegend Wasserstoff, Methan, Ethan und Ethylen und wahlweise weitere Kohlenwasserstoffe enthält, unter Verwendung einer Destillationssäule und einer Entspannungsturbine aus. Bei dem Gasgemisch handelt es sich beispielsweise um das bereits erläuterte Spaltgas.

In dem Gasgemisch liegt das Verhältnis von Methan zu Wasserstoff beispielsweise bei 0,5 bis 2, insbesondere bei 1,2 bis 1,4, und/oder das Verhältnis von Methan zu C2-Kohlenwasserstoffen beispielsweise bei 0,03 bis 0,08, insbesondere bei 0,04 bis 0,06. Diese Werte sind typisch für Spaltgase, die beim Dampfspalten ethanreich(er)er Frischeinsätze erhalten werden. Beim Dampfspalten von Naphtha als Frischeinsatz gebildete Spaltgase enthalten hingegen beispielsweise Methan und Wasserstoff im Verhältnis von 10 bis 15, insbesondere von 12 bis 13, und/oder Methan und C2-Kohlenwasserstoffe im Verhältnis von 0,3 bis 0,6, insbesondere von 0,4 bis 0,5.

Das Gasgemisch wird von einem ersten Temperaturniveau, beispielsweise Umgebungstemperatur oder darüber (aufgrund von Verdichtungswärme) auf ein zweites Temperaturniveau, das zwischen den Kondensationstemperaturen von Ethylen und Methan bei dem verwendeten Druckniveau liegt, abgekühlt, wobei zumindest ein Kondensat aus dem Gasgemisch abgeschieden wird.

Dies erfolgt herkömmlicherweise dadurch, dass ein entsprechendes Gasgemisch sukzessive durch mehrere Wärmetauscher, beispielsweise Plattenwärmetauscher, geführt wird und sich dabei abkühlt. Stromab jedes der Wärmetauscher ist ein Flüssigkeitsabscheider vorgesehen, in dem jeweils ein Kondensat erhalten wird. Die in den Flüssigkeitsabscheidern gasförmig verbleibenden Anteile werden jeweils durch den nächsten Wärmetauscher geführt und anschließend in einen weiteren Flüssigkeitsabscheider eingespeist.

Vorteilhafterweise wird das Gasgemisch also in mehreren Stufen abgekühlt, wobei auf zumindest zwei der Stufen Kondensate abgeschieden werden, aus denen der zumindest eine überwiegend Methan, Ethan und/oder Ethylen enthaltende Strom gebildet wird. Eine derartige stufenweise Abkühlung erlaubt die Verwendung herkömmlicher Wärmetauscher und eine energetisch besonders effektive Gestaltung einer entsprechenden Anlage.

Aus dem zumindest einen Kondensat wird zumindest ein überwiegend Methan, Ethan und/oder Ethylen enthaltender Strom gebildet und zumindest teilweise in die Destillationssäule eingespeist.

Bei stufenweiser Abkühlung können sämtliche erhaltenen Kondensate zumindest teilweise in die Destillationssäule eingespeist werden. Die Kondensate enthalten dabei, je nach ihrer Abscheidetemperatur, unterschiedliche Gehalte von Kohlenwasserstoffen mit unterschiedlicher Kondensationstemperatur. Die Kondensate werden daher in die verwendete Destillationssäule in unterschiedlichen Höhen eingespeist, wobei typischerweise Entspannungsventile verwendet werden.

Die aus dem oder den Kondensaten gebildeten Ströme, die in die Destillationssäule eingespeist werden, können auch unter Abtrennung weiterer Komponenten, also in Form "abgeleiteter" Ströme im obigen Sinn, gebildet werden. Beispielsweise können Kohlenwasserstoffe mit drei und mehr Kohlenstoffatomen abgetrennt werden.

In der Destillationssäule wird ein überwiegend Methan und Wasserstoff und ferner Ethylen enthaltendes Kopfprodukt erzeugt und zumindest teilweise als Kopfstrom aus der Destillationssäule abgezogen. Ziel der Erfindung ist es, wie erwähnt, das in dem Kopfprodukt enthaltene Ethylen möglichst weitgehend zurückzugewinnen. Nach einem weiteren Abkühlen wird aus dem Kopfstrom zumindest eine ethylenangereicherte Flüssigkeit abgeschieden und zumindest teilweise als Rücklaufstrom auf die Destillationssäule aufgegeben. Herkömmlicherweise wird der Kopfstrom beispielsweise mit einem Kältemittel bei -95 °C abgekühlt, also beispielsweise einem Kältemittel auf einem Temperaturniveau zwischen den Kondensationstemperaturen von Ethylen und Methan bei dem vorherrschenden Druck.

Erfindungsgemäß ist vorgesehen, das Abkühlen des Kopfstroms zumindest teilweise mittels eines Teilstroms eines auf dem zweiten Temperaturniveau (zwischen den Kondensationstemperaturen von Ethylen und Methan) gasförmig verbleibenden Anteils des eingesetzten Gasgemischs durchzuführen. Mit anderen Worten wird hierbei also ein Gasgemisch verwendet, das nach dem Abscheiden des wenigstens einen Kondensats verbleibt. Dieser Teilstrom wird ferner anschließend zumindest teilweise entspannt, wodurch zusätzliche Kälte erzeugt werden kann. Die Entspannung erfolgt vorzugsweise in einer oder mehreren Entspannungsturbinen. Zusätzlich kann der auch nach der weiteren Abkühlung nicht kondensierte Anteil des Kopfproduktes der Destillationssäule ebenfalls über diese Entspannungsturbine geleitet werden, um die zusätzliche Kälte zu maximieren. Der Mengenstrom des zum Abkühlen des Kopfstroms verwendeten Teilstroms kann in Abhängigkeit von einem Wasserstoffgehalt, einem Methangehalt und/oder einem Methan-Wasserstoff-Verhältnis des Gasgemischs eingestellt werden, um das zweite Temperaturniveau zu minimieren.

Bei dem auf dem zweiten Temperaturniveau (zwischen den Kondensationstemperaturen von Ethylen und Methan) gasförmig verbleibenden Anteil des Gasgemischs handelt es sich um ein Gasgemisch, das überwiegend Wasserstoff und Methan aufweist, wobei das Verhältnis von Wasserstoff und Methan sich aus den zuvor erwähnten Gehalten ableitet. Ein gewisser Anteil des Methans ist dabei jedoch bereits in das wenigstens eine Kondensat übergegangen.

In dem erfindungsgemäßen Verfahren wird vorteilhafterweise aus dem auf dem zweiten Temperaturniveau (zwischen den Kondensationstemperaturen von Ethylen und Methan) gasförmig bleibenden Anteil des Gasgemischs ein weiterer Teilstrom gebildet, der auf ein drittes Temperaturniveau, das 10 K bis 50 K, beispielsweise 20 K, 30 K oder 40 K, unterhalb des zweiten Temperaturniveaus liegt, abgekühlt und anschließend zur Bildung eines methanreichen Stroms und eines wasserstoffreichen Stroms verwendet wird. Das Methan wird typischerweise aus einer entsprechenden Anlage ausgeleitet und als Brenngas verwendet, wohingegen der Wasserstoff vorteilhafterweise für andere Zwecke, beispielsweise in einem Synthesegas oder für Hydrierungszwecke, eingesetzt werden kann.

Die erläuterte Abkühlung erfolgt vorteilhafterweise auf einem Druckniveau von 10 bar bis 50 bar, insbesondere von 20 bis 40 bar. Der Teilstrom, der zum Abkühlen des Kopfstroms verwendet wird, wird anschließend (ggf. zusammen mit dem beim weiteren Abkühlen gasförmig verbliebenen Anteil des Kopfstroms) zumindest teilweise auf ein Druckniveau von 5 bar bis 10 bar, insbesondere von 6 bar bis 8 bar, entspannt. Die genannten Drücke erweisen sich als energetisch besonders günstig und bewirken einen effektiven Wärmeaustausch in den verwendeten Wärmetauschern.

In einem erfindungsgemäßen Verfahren wird der Kopfstrom vorteilhafterweise in mehreren Stufen abgekühlt, wobei auf zumindest zwei Stufen ethylenreiche Flüssigkeiten abgeschieden und zu dem Rücklaufstrom vereinigt werden. Insgesamt kann damit Ethylen nahezu vollständig aus dem Kopfstrom zurückgewonnen werden. Hierbei können neben dem Teilstrom des auf dem zweiten Temperaturniveau (zwischen den Kondensationstemperaturen von Ethylen und Methan) gasförmig verbleibenden Anteils des Gasgemischs ein oder mehrere Kältemittel, beispielsweise C2-Kältemittel, eingesetzt werden, die die benötigten Temperaturen aufweisen.

Erfindungsgemäß ist also vorgesehen, nicht den gesamten auf dem zweiten Temperaturniveau (zwischen den Kondensationstemperaturen von Ethylen und Methan) gasförmig verbleibenden Anteil des Gasgemischs in einen methanreichen Strom und einen wasserstoffreichen Strom zu trennen und unmittelbar aus der Anlage auszuführen. Vielmehr wird hieraus ein Teilstrom gebildet, der zum weiteren Kühlen des Kopfstroms der Destillationssäule verwendet und danach entspannt wird. Hierdurch kann eine verringerte Menge von Methan am Kopf der Trennsäule ausgeglichen werden. Die Entspannung ermöglicht aufgrund der zusätzlich bereitgestellten Kälte eine ausreichende Trennung von Methan und Wasserstoff von Ethylen beim Abscheiden des zumindest einem Kondensates aus dem Gasgemisch und erhöht die Kondensation von Methan zur Destillationssäule. Aufgrund der oben erwähnten Teilentspannung liegen die Ströme noch bei ausreichendem Druck vor, um beispielsweise als Druckprodukt ausgeführt werden und als Kältemittel in den Wärmetauschern dienen zu können.

In dem erfindungsgemäßen Verfahren wird also vorteilhafterweise ferner ein beim stufenweisen Abkühlen des Kopfstroms gasförmig verbleibender Anteil des Kopfstroms ebenfalls zumindest teilweise in derselben Apparatur entspannt, so dass weitere Kälte erzeugt werden kann. Dies ist auch deshalb möglich, weil im Rahmen der vorliegenden Erfindung der Kopfstrom mit dem bei dem zweiten Temperaturniveau gasförmig verbliebenen Anteil des Gasgemischs abgekühlt und dieses seinerseits erwärmt wird. Es kann daher mit dem gasförmig verbleibenden Anteil des Kopfstroms vereinigt werden, ohne dass dieser aufgrund der ohne entsprechende Erwärmung sehr viel tieferen Temperatur des bei dem zweiten Temperaturniveau gasförmig verbliebenen Anteils des Gasgemischs partiell kondensiert würde. Ein partiell kondensierter Strom könnte nicht in üblichen Entspannungsturbinen entspannt werden.

Vorzugsweise liegen der entspannte gasförmig verbliebene Anteil des Kopfstroms und der in derselben Anordnung entspannte, bei dem zweiten Temperaturniveau gasförmig verbliebene Anteil des Gasgemischs als Zweiphasenstrom vor. Er enthält damit Flüssigkeit, die in den zum Abkühlen des Gasgemischs verwendeten Plattenwärmetauschern verdampft werden kann (latente Kälte).

Wie bereits mehrfach erläutert, eignet sich die vorliegende Erfindung insbesondere für Verfahren, bei denen als Gasgemisch ein Spaltgas eines Dampfspaltverfahrens oder ein aus einem solchen Spaltgas abgeleiteter Strom verwendet wird, insbesondere bei ethanreich(er)en Frischeinsätzen.

Insbesondere erlaubt das Verfahren die gezielte Einstellung eines Mengenstroms des zum Abkühlen des Kopfstroms verwendeten Teilstroms in Abhängigkeit von einem Wasserstoffgehalt, einem Methangehalt und/oder einem Methan-Wasserstoff-Verhältnis des Gasgemischs. Hierdurch lässt sich das erfindungsgemäße Verfahren beliebig an entsprechende Methan- bzw. Wasserstoffgehalte anpassen. Der Mengenstrom des zum Abkühlen des Kopfstroms verwendeten Teilstroms wird gezielt eingestellt um das zweite Temperaturniveau zu reduzieren und damit die Produktverluste zum Brenngas in der Anlage zu minimieren. Des Weiteren kann mit der Einstellung des zweiten Temperaturniveaus durch den Teilstrom die Menge an Methan zur Destillationskolonne erhöht werden.

Eine Trennanlage, die zur Trennung eines Gasgemischs, das überwiegend Wasserstoff, Methan, Ethan und Ethylen und wahlweise weitere Kohlenwasserstoffe enthält, eingerichtet ist, und die eine Destillationssäule aufweist, ist ebenfalls Gegenstand der Erfindung. Diese Anlage weist Mittel auf, die dafür eingerichtet sind, zum weiteren Abkühlen des Kopfstroms zumindest einen Teilstrom eines auf dem Temperaturniveau zwischen den Kondensationstemperaturen von Ethylen und Methan gasförmig bleibenden Anteils des Gasgemischs zu verwenden, wobei Mittel vorgesehen sind, die dafür eingerichtet sind, den Teilstrom nach der Verwendung zum Abkühlen des Kopfstroms zumindest teilweise zu entspannen.

Eine entsprechende Trennanlage ist insbesondere für ein Verfahren eingerichtet, wie es zuvor erläutert wurde. Auf die genannten Vorteile wird daher verwiesen.

Auch ein Verfahren zum Umrüsten einer Trennanlage ist Gegenstand der vorliegenden Erfindung. Das Umrüsten umfasst insbesondere das Bereitstellen von Mitteln, die dafür eingerichtet sind, aus dem nach dem Abscheiden der Kondensate auf dem ersten Druckniveau und dem zweiten Temperaturniveau gasförmig bleibenden Anteil des Gasgemischs einen Teilstrom zu bilden, den Teilstrom zum weiteren Kühlen eines nach einer ersten Abkühlung und einer ersten Phasentrennung gasförmig verbliebenen Anteils eines Kopfprodukts der Destillationssäule zu verwenden, und den Teilstrom sowie den nach der weiteren Abkühlung gasförmig verbleibenden Kopfstrom danach gemeinsam zu entspannen.

Kurze Beschreibung der Zeichnungen
Figur 1 zeigt ein Verfahren zur Erzeugung von Kohlenwasserstoffen gemäß dem Stand der Technik in Form eines schematischen Ablaufplans.
Figuren 2A, 2B und 2C zeigen eine Trennanlage gemäß einer Ausführungsform der Erfindung in Form eines Prozessflussdiagramms.

### Ausführliche Beschreibung der Zeichnungen

In den Figuren sind einander entsprechende Elemente mit identischen Bezugszeichen angegeben und werden der Übersichtlichkeit halber nicht wiederholt erläutert.

In Figur 1 ist der Ablauf eines Verfahrens zur Erzeugung von Kohlenwasserstoffen gemäß dem Stand der Technik in Form eines schematischen Ablaufplans dargestellt. Es ist ein Dampfspaltprozess S vorgesehen, der unter Verwendung eines oder mehrerer Spaltöfen S1 bis S3 durchgeführt werden kann. Nachfolgend wird nur der Betrieb des Spaltofens S3 erläutert, die weiteren Spaltöfen S1 und S2 können in entsprechender Weise arbeiten oder wegfallen.

Der Spaltofen S3 wird mit einem Strom A als Ofeneinsatz beschickt, bei dem es sich zumindest teilweise um einen sogenannten Frischeinsatz handeln kann, der aus anlagenexternen Quellen zur Verfügung gestellt wird, und zu einem Teil um einen sogenannten Recyclestrom, der in dem Verfahren selbst gewonnen wird, wie unten erläutert. Auch die anderen Spaltöfen S1 und S2 können mit entsprechenden Strömen beschickt werden. Unterschiedliche Ströme können auch in unterschiedliche Spaltöfen S1 bis S3 eingespeist werden, ein Strom A kann auf mehrere Spaltöfen aufgeteilt werden oder mehrere Teilströme können zu einem Sammelstrom vereinigt werden, der als Strom A einem der Spaltöfen S1 bis S3 zugeführt wird.

Durch Dampfspalten in dem Dampfspaltprozess S wird ein Rohgasstrom B erhalten, der bisweilen bereits an dieser Stelle als Spaltgasstrom bezeichnet wird. Der Rohgasstrom B wird in einer Reihe nicht dargestellter Aufbereitungsstufen eines Aufbereitungsprozesses 20 aufbereitet, beispielsweise einem sogenannten Ölquench unterzogen, vorfraktioniert, verdichtet, weiter gekühlt und getrocknet.

Der entsprechend behandelte Strom B, das eigentliche Spaltgas, das nun mit C bezeichnet ist, wird anschließend einem Trennprozess 30 unterworfen. In diesem wird eine Anzahl von Fraktionen gewonnen, die hier, wie eingangs erläutert, entsprechend der Kohlenstoffanzahl der überwiegend enthaltenen Kohlenwasserstoffe bezeichnet werden. Der in der Figur 1 dargestellte Trennprozess 30 arbeitet nach dem bekannten Prinzip "Deethanizer First".

Dem Fachmann sind, beispielsweise aus dem eingangs erwähnten Artikel "Ethylene" in Ullmann's Encyclopedia of Industrial Chemistry, mehrere Verfahrensalternativen bekannt, die sich insbesondere in der Aufbereitung des Spaltgases C und/oder dem verwendeten Trennprozess unterscheiden.

In dem Trennprozess 30 wird dabei aus dem Spaltgas C zunächst in einer Trenneinrichtung 31 eine C2minus-Fraktion gasförmig abgetrennt, die überwiegend Methan, Ethan, Ethylen und Acetylen und insbesondere auch noch Wasserstoff enthalten kann. Die C2minus-Fraktion wird insgesamt einem Hydrotreatmentprozess 41 unterzogen, um enthaltenes Acetylen zu Ethylen umzusetzen. Anschließend werden aus der C2minus-Fraktion in einer C2minus-Trenneinrichtung 32 Methan CH4 und Wasserstoff H2 in beliebiger Reihenfolge nacheinander oder gemeinsam abgetrennt und beispielsweise als Brenngas verwendet.

Es verbleibt eine C2-Fraktion, die in einer C2-Trenneinrichtung 33 in Ethylen C2H4 und Ethan C2H6 aufgetrennt wird. Letzteres kann auch als Recyclestrom D in einem oder mehreren Spaltöfen S1 bis S3 erneut dem Dampfspaltprozess S unterworfen werden. Im dargestellten Beispiel werden die Recycleströme D und E zu dem Strom A zugegeben. Die Recycleströme D und E und der Strom A können auch in unterschiedliche Spaltöfen S1 bis S3 geführt werden.

In der Trenneinrichtung 31 verbleibt eine flüssige C3plus-Fraktion, die in eine Trenneinrichtung 34 (den sogenannten Depropanizer) überführt wird. In der Trenneinrichtung 34 wird aus der C3plus-Fraktion eine C3-Fraktion abgetrennt und einem Hydrotreatmentprozess 42 unterzogen, um in der C3-Fraktion enthaltenes Methylacetylen zu Propylen umzusetzen. Anschließend wird die C3-Fraktion in einer C3-Trenneinrichtung 36 in Propylen C3H6 und Propan C3H8 aufgetrennt. Letzteres kann als Recyclestrom E in einem oder mehreren Spaltöfen S1 bis S3, separat oder mit anderen Strömen, erneut dem Dampfspaltprozess S unterworfen werden.

In der Trenneinrichtung 14 verbleibt eine flüssige C4plus-Fraktion, die in eine Trenneinrichtung 35 (den sogenannten Debutanizer) überführt wird. In der Trenneinrichtung 35 wird aus der C4plus-Fraktion eine C4-Fraktion gasförmig abgetrennt. Es verbleibt eine flüssige C5plus-Fraktion.

Es versteht sich, dass sämtliche der dargestellten Fraktionen auch geeigneten Nachbehandlungsschritten unterworfen werden können. Beispielsweise kann aus der C4-Fraktion 1,3-Butadien abgetrennt werden. Es können ferner zusätzliche Recycleströme verwendet werden, die analog zu den Recycleströmen D und E dem Dampfspaltprozess 10 unterworfen werden können.

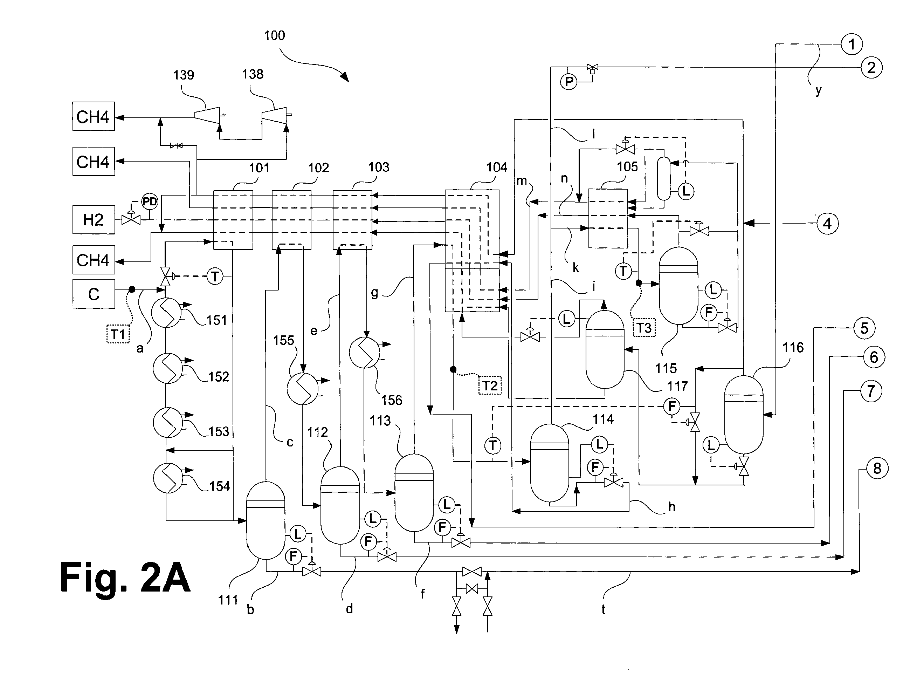

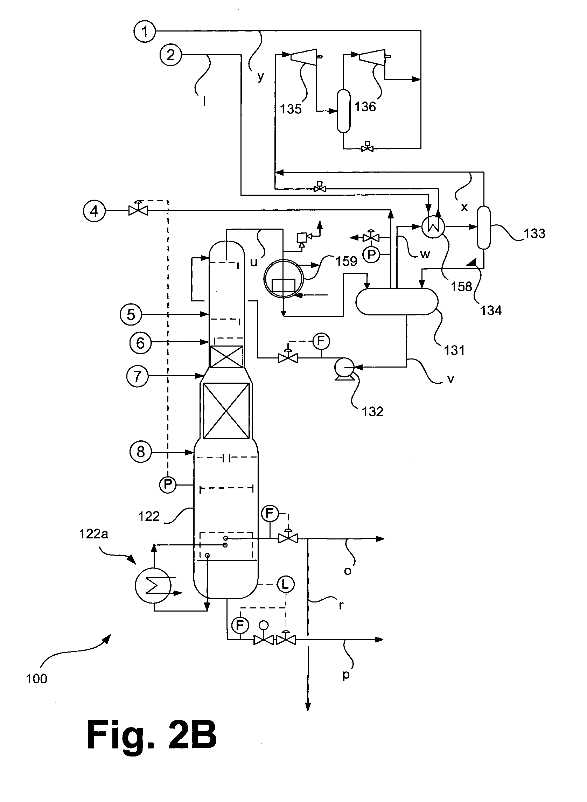

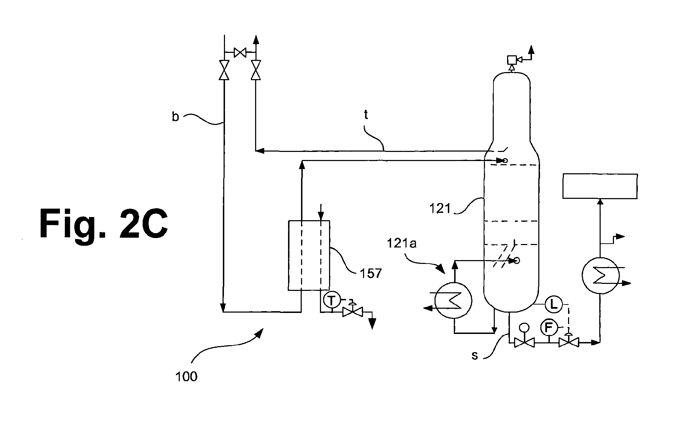

In den Figuren 2A, 2B und 2C ist eine Trennanlage gemäß einer Ausführungsform der Erfindung in Form eines Prozessflussdiagramms dargestellt. Die Figuren 2A und 2B zeigen dabei Komponenten, die über die Verknüpfungspunkte 1 bis 8 miteinander verbunden sind. Die in Figur 2A und 2C dargestellten Komponenten sind in Form der Ströme b und t miteinander verknüpft. Die in den Figuren 2A, 2B und 2C getrennt gezeigten Anlagenteile können insbesondere auch baulich zusammengefasst sein und sind hier aus Gründen der Übersichtlichkeit in zwei getrennten Figuren veranschaulicht.

In den Figuren 2A und 2B ist eine Reihe von Ventilen und Regeleinrichtungen veranschaulicht, die nicht im Detail erläutert werden, deren Darstellung jedoch fachüblich ist und deren Funktion sich daher für den Fachmann unmittelbar aus den Figuren 2A und 2B ergibt. Bei den dargestellten Regeleinrichtungen bezeichnen beispielsweise die Buchstaben L einen Füllstandsregler, F einen Flussregler, T einen Temperaturregler und P einen Druckregler.

Der Trennanlage, die in den Figuren 2A, 2B und 2C insgesamt mit 100 bezeichnet ist, wird ein Gasgemisch C zugeführt, bei dem es sich um das mehrfach erwähnte und auch in der Figur 1 gezeigte Spaltgas handeln kann. Das Gasgemisch C, das neben Wasserstoff im Wesentlichen Kohlenwasserstoffe mit zwei und gegebenenfalls auch mehr Kohlenstoffatomen und Methan enthält, wird als Strom a auf einem definierten ersten Temperaturniveau, hier mit T1 bezeichnet, der Anlage 100 zugeführt. Es liegt an dem mit T1 bezeichneten Punkt ferner auf einem definierten Druckniveau vor, das hier als erstes Druckniveau bezeichnet wird, jedoch nicht gesondert veranschaulicht ist.

Der Strom a wird je nach Bedarf anteilig in einem ersten Wärmetauscher 101 und weiteren Wärmetauschern 151 bis 154 abgekühlt. Der Betrieb des ersten Wärmetauschers 101 wird unten erläutert. Im Wesentlichen stammt die in dem ersten Wärmetauscher 101 bereitgestellte Kälte aus Fluidströmen, die aus dem Gasgemisch C (also Strom a) gebildet werden. Entsprechendes gilt auch für unten erläuterte zweite bis vierte Wärmetauscher 102 bis 104. Die weiteren Wärmetauscher 151 bis 154 sowie zusätzliche in der Trennanlage 100 vorhandene Wärmetauscher, beispielsweise die Wärmetauscher 155 und 156, werden mit geeigneten Kältemitteln betrieben, beispielsweise mit unter Bezugnahme auf die Figur 1 erläuterten Kohlenwasserstofffraktionen.

Der Strom a wird nach der Abkühlung auf einem entsprechend einstellbaren Temperaturniveau in einen ersten Flüssigkeitsabscheider 111 eingespeist. In dem Flüssigkeitsabscheider 111 wird aus dem Strom a ein Kondensat abgeschieden und als Strom b abgezogen. Der Strom b kann noch Kohlenwasserstoffe mit drei und mehr Kohlenstoffatomen enthalten, die in einer Anordnung, wie sie in Figur 2C gezeigt ist, abgetrennt werden können. Ein entsprechend behandelter Strom wird hier mit t bezeichnet. Enthält der Strom b keine Kohlenwasserstoffe mit drei und mehr Kohlenstoffatomen, kann der Strom b dem Strom t entsprechen. Der Strom t wird über den Verknüpfungspunkt 8 in eine Trennsäule 122 eingespeist (vgl. Figur 2B).

Kopfseitig des Flüssigkeitsabscheiders 111 abgezogenes Fluid wird in Form eines Stroms c durch den zweiten Wärmetauscher 102 und den zusätzlichen Wärmetauscher 155 geführt und weiter abgekühlt. Der Strom c wird anschließend in einen zweiten Flüssigkeitsabscheider 112 eingespeist. Aus einem in dem zweiten Flüssigkeitsabscheider 112 erhaltenen Kondensat wird der Strom d gebildet und über den Verknüpfungspunkt 7 wie bereits der Strom t ebenfalls in die Trennsäule 122 (siehe Figur 2B) eingespeist.

In dem zweiten Flüssigkeitsabscheider 112 gasförmig verbliebenes Fluid des Stroms c und damit des Gasgemischs C wird durch den dritten Wärmetauscher 103 und den zusätzlichen Wärmetauscher 156 geführt und damit nochmals abgekühlt. Dieser Strom, hier mit e bezeichnet, wird in einen dritten Flüssigkeitsabscheider 113 eingespeist, in dem wiederum ein Kondensat erhalten und in Form des Stroms f abgezogen wird. Auch dieser Strom f wird, über Verknüpfungspunkt 6, in die Destillationssäule 122 (siehe Figur 2B) eingespeist.

Ein auch in dem dritten Flüssigkeitsabscheider 113 gasförmig verbliebener Anteil des Stroms e und damit des Gasgemischs C wird als Strom g in einem vierten Wärmetauscher 104 abgekühlt und liegt stromab dieses vierten Wärmetauschers 104 auf einem zweiten Temperaturniveau vor, das zwischen den Kondensationstemperaturen von Ethylen und Methan liegt und in Figur 2 mit T2 bezeichnet ist.

Auf dem zweiten Temperaturniveau T2 zwischen den Kondensationstemperaturen von Ethylen und Methan und im Wesentlichen noch auf dem ersten Druckniveau, auf dem der Strom C in die Trennanlage 100 eingespeist wurde, wird der Strom g in einen vierten Flüssigkeitsabscheider 114 überführt. Ein hier erhaltenes Kondensat h wird in dem vierten Wärmetauscher 104 erwärmt und anschließend über Verknüpfungspunkt 5, wie zuvor die Kondensate t, d und f über die Verknüpfungspunkte 8, 7 und 6, in die Destillationssäule 122 (siehe Figur 2B) eingespeist.

Ein kopfseitig des Flüssigkeitsabscheiders 114 abgezogener Strom i, also ein auch in dem vierten Flüssigkeitsabscheider 114 und damit zwischen den Kondensationstemperaturen von Ethylen und Methan gasförmig verbliebener Anteil des Gasgemischs C, wird in zwei Teilströme k und l aufgeteilt. Da der Strom g auf dem genannten zweiten Temperaturniveau T2, beispielsweise bei -130 °C, in den vierten Flüssigkeitsabscheider 114 überführt wird und da das erste Druckniveau bei ca. 30 bar liegt, besteht der Strom i im Wesentlichen noch aus Wasserstoff und Methan, deren Kondensationstemperaturen bei dem vorliegenden Druck unterhalb von -130 °C liegen. Ethylen, dessen Kondensationstemperatur bei dem vorliegenden Druck deutlich oberhalb von -130°C liegt, kann teilweise durch die starke Präsenz von Wasserstoff nicht kondensiert werden und ein Anteil des enthaltenen Ethylens wird in den Strom i überführt. Damit ist das zweite Temperaturniveau T2 entscheidend dafür, wie groß der Anteil an Ethylen ist, welcher in das Gasgemisch des Stroms i gelangt. Wie zuvor ausführlich beschrieben, kann dieses Temperaturniveau T2 positiv beeinflusst werden (um Ethylenverluste zu minimieren) durch die teilweise Entspannung des Teilstroms I des gasförmigen verbleibenden Anteil des Stroms g.

Insgesamt wird damit das Gasgemisch C auf ein zweites Temperaturniveau T2 zwischen den Kondensationstemperaturen von Ethylen und Methan abgekühlt, wobei im dargestellten Beispiel mehrere Kondensate aus dem Gasgemisch C abgeschieden werden. Es kann jedoch alternativ zur Darstellung in Figur 2A auch beispielsweise eine einstufige Abkühlung unter Erhalt nur eines Kondensats erfolgen. Im dargestellten Beispiel werden aus den erhaltenen Kondensaten insgesamt vier überwiegend Methan, Ethan und/oder Ethylen enthaltende Ströme t, d, f und h gebildet und zumindest teilweise in die Destillationssäule eingespeist. Der Strom t kann auch aus dem Kondensat aus dem ersten Flüssigkeitsabscheider 111 bzw. einem entsprechenden Strom b durch Abscheiden höherer Kohlenwasserstoffe "gebildet" werden, wie in Figur 2C im Detail veranschaulicht.

Der Teilstrom k wird durch den fünften Wärmetauscher 105 geführt und dort auf ein drittes Temperaturniveau abgekühlt, das hier mit T3 bezeichnet ist und 10 K bis 50 K unterhalb des zweiten Temperaturniveaus, beispielsweise bei -160 °C, liegt. Der Teilstrom k wird anschließend in einen fünften Flüssigkeitsabscheider 115 eingespeist. Ein Kondensat und ein gasförmig verbliebener Anteil des Teilstroms k aus dem fünften Flüssigkeitsabscheider 115 können je nach Bedarf miteinander vereinigt und/oder in unterschiedlichen Anteilen in dem fünften Wärmetauscher 105 erwärmt werden, wobei auch eine vor- und/oder nachgeschaltete Phasentrennung erfolgen kann. Im Ergebnis werden aus dem Kondensat und dem gasförmig verbliebenen Anteil aus dem fünften Flüssigkeitsabscheider 115 die Ströme m und n erhalten. Hierbei kann es sich im regulären Betrieb der Trennanlage 100 bei dem Strom n um einen wasserstoffreichen Strom handeln. Bei dem Strom m kann es sich hingegen im regulären Betrieb der Trennanlage 100 im Wesentlichen um Methan bzw. einen methanreichen Strom handeln. Die Ströme m und n werden in dem vierten, dem dritten, dem zweiten und dem ersten Wärmetauscher 104, 103, 102 und 101 erwärmt und können aus der Anlage ausgeführt werden. Die in der Figur 2A gezeigten Zwischenverbindungen zwischen den Strömen m und n, insbesondere auch die bereits stromauf des fünften Wärmetauschers 105 vorgesehenen Zwischenverbindungen, können insbesondere beim Anfahren einer entsprechenden Anlage 100 und/oder zur Einstellung der Gehalte der Ströme m und n an Methan bzw. Wasserstoff verwendet werden.

Mit anderen Worten wird der Teilstrom k zur Bildung eines wasserstoffreichen Stroms n und eines methanreichen Stroms m verwendet. Wie zuvor mehrfach erläutert, wird bei der Verwendung von Frischeinsätzen, die größere Mengen an Ethan enthalten, beim Dampfspalten eine vergleichsweise große Menge Wasserstoff (nämlich, wie erwähnt, die zwei- bis vierfache Menge), aber auch erheblich weniger Methan (typischerweise, wie erwähnt, weniger als 50 % der beim Dampfspalten von schwereren Frischeinsätzen erhaltenen Menge) gebildet.

Die Folge der Änderung der absoluten Mengen und Konzentrationen ist, dass bestehende Zerlegungsanlagen nicht mehr in der Lage sind, die Ethylenverluste in die C1-Fraktion des Spaltgases auf den gewünschten bzw. ursprünglichen Wert oder, anders ausgedrückt, auf einen spezifikationsgerechten Wert, zu begrenzen. Dies wird unter Bezugnahme auf den Kopfstrom u aus der Trennsäule 122 zu Figur 2B erläutert. Der genannte Umstand ist darauf zurückzuführen, dass sich das Mengenverhältnis von Methan zu den Kohlenwasserstoffen mit zwei Kohlenstoffatomen (also ein C1/C2-Mengenverhältnis) in den in die Trennsäule 122 geführten Strömen (hier also den Strömen t, d, f und h) deutlich verringert. Hierdurch gerät der obere Bereich der Trennsäule 122 in Unterlast, und die Kondensation im Kopfkondensator der Trennsäule 122 wird erschwert. Dies führt zu erhöhten Ethylenverlusten. Vereinfacht und stofflich ausgedrückt ist die Methanmenge zu gering, um genügend Kälte auf ausreichend tiefem Temperaturniveau zu erzeugen.

Die Erfindung schlägt daher die Aufteilung des Stroms i in die bereits erläuterten Teilströme I und k vor. Der Teilstrom k wird dabei, wie zuvor erläutert, im Wesentlichen wie in herkömmlichen Trennanlagen auch behandelt. Ein wesentlicher Aspekt der vorliegenden Erfindung ist hingegen die Behandlung des Teilstroms I. Dieser wird über den Verknüpfungspunkt 2 geführt und als Kühlmittel in einem Wärmetauscher 158 verwendet. Durch die Verwendung des Wärmetauschers 158 können die zuvor erläuterten Ethylenverluste an der Trennsäule 122 beträchtlich reduziert werden. Zur weiteren Erläuterung wird im Folgenden auf die Figur 2B Bezug genommen.

Zentrale Komponente des in Figur 2B gezeigten Anlagenteils ist die mehrfach erwähnte Trennsäule 122, der die Kondensate aus dem ersten bis vierten Flüssigkeitsabscheider 111 bis 114 zugeführt werden (vgl. Verknüpfungspunkte 5 bis 8 in den Figuren 2A und 2B).

Die Trennsäule 122 wird mit einem Sumpfverdampfer 122a betrieben, der mit einem geeigneten Heizmedium beheizt werden kann. Aus einem unteren Bereich der Trennsäule 122 werden beispielsweise die Ströme o und p abgezogen und in weitere Trenneinrichtungen überführt, wie hier nicht näher erläutert. Ein Strom r kann beispielsweise beim Anfahren der Trennanlage 100 verwendet und mit dem Strom s (vgl. Figur 2C) vereinigt werden.

Vom Kopf der Trennsäule 122 wird ein Kopfstrom u eines entsprechenden Kopfprodukts abgezogen und in einem Wärmetauscher 159, der beispielsweise mit C2-Kältemittel mit -95 °C betrieben werden kann, abgekühlt. Der Wärmetauscher 159 kann auch in herkömmlichen Anlagen 100 vorhanden sein. Das in dem Wärmetauscher 159 verwendete Kältemittel liegt also auf einem Temperaturniveau zwischen den Kondensationstemperaturen von Ethylen und Methan vor. Ziel ist es, Ethylen aus dem Kopfstrom u weitgehend abzuscheiden und als flüssigen Rücklaufstrom v erneut auf die Trennsäule 122 aufzugeben. Hierzu wird der abgekühlte Strom u in einem Flüssigkeitsabscheider 131 überführt. Ein Kondensat aus dem Flüssigkeitsabscheider 131 wird als Rücklaufstrom v mittels einer Pumpe 132 am Kopf der Trennsäule 122 erneut aufgegeben.

Wie zuvor erläutert, ist insbesondere die Bildung dieses Kondensats v bei einer Erhöhung des Wasserstoffanteils in den in die Trennsäule 122 eingespeisten Strömen t, d, f und h erschwert. Daher wird ein in dem Flüssigkeitsabscheider 131 gasförmig verbliebener Anteil, hier mit w bezeichnet, erfindungsgemäß durch den bereits erwähnten zusätzlichen Wärmetauscher 158 geführt, der mit dem Teilstrom I gekühlt wird. Damit kann in einem weiteren Flüssigkeitsabscheider 133 ein größerer Anteil einer Flüssigfraktion abgeschieden werden, wie mit Symbol 134 veranschaulicht. Dieser größere Anteil kann wieder in den Flüssigkeitsabscheider 131 überführt und damit letztlich als Rücklaufstrom v auf die Trennsäule 122 aufgegeben werden. Die Ethylenverluste in den Kopfstrom x des Flüssigkeitsabscheiders 133 verringern sich hierdurch beträchtlich. Der Flüssigkeitsabscheider 133 kann erfindungsgemäß über dem Flüssigkeitsabscheider 131 installiert werden um eine kalte Pumpe zu sparen und die gebildete Flüssigkeit vom Abscheider 133 durch das natürliche Gefälle zu befördern.

Nachdem der Strom I durch den Wärmetauscher 158 geführt wurde, wird er mit dem Strom x vereinigt und in zwei Entspannungsturbinen 135 und 136 entspannt. Der entspannte zweiphasige Strom y mit einer Kondensationstemperatur zwischen Methan und Wasserstoff wird über den Verknüpfungspunkt 1 in den in der Figur 2A gezeigten Anlagenteil zum Flüssigkeitsabscheider 116 geführt.

Der gasförmige Anteil des Stroms y wird in den Wärmetauschern 104 bis 101 erwärmt und in den Verdichtern 138 und 139 verdichtet. Der aus dem in Figur 2B gezeigten Anlagenteil überführte Strom y wird damit letztlich zur Bildung von Methanfraktionen verwendet, die bei unterschiedlichen Drücken aus der Trennanlage 100 ausgeführt werden können.

In Figur 2C ist die Behandlung eines Stroms b (vgl. Figur 2A) veranschaulicht, wenn dieser noch Kohlenwasserstoffe mit drei und mehr Kohlenstoffatomen enthält. Im Normalbetrieb der Trennanlage 100 wird das Kondensat b dabei zunächst durch einen Wärmetauscher 157 geführt und danach in eine Trennsäule 121 eingespeist. In der Trennsäule 121, die mit einem Sumpfverdampfer 121 a betrieben wird, beispielsweise unter Verwendung von Heißwasser oder Dampf, wird aus dem Kondensat b ein Sumpfprodukt s abgeschieden, welches aus der Trennanlage 100 ausgeführt werden kann. Bei dem Sumpfprodukt s aus der Destillationssäule 121 handelt es sich insbesondere um Kohlenwasserstoffe, die bei Temperaturen von unter -30 °C ausfrieren würden. Ein in einem Kopfbereich der Trennsäule 121 anfallendes Fluid wird (vgl. Figur 2A) als Strom t aus der Trennsäule 121 abgezogen und über den Verknüpfungspunkt 8 in eine Trennsäule 122 eingespeist (vgl. Figur 2B).

## Patentansprüche

1. Verfahren zur Trennung eines Gasgemischs (C), das überwiegend Wasserstoff, Methan, Ethan und Ethylen und wahlweise weitere Kohlenwasserstoffe enthält, unter Verwendung einer Destillationssäule (122), bei dem
- das Gasgemisch (C) von einem ersten Temperaturniveau (T1) auf ein zweites Temperaturniveau (T2), das zwischen den Kondensationstemperaturen von Ethylen und Methan liegt, abgekühlt wird, wobei zumindest ein Kondensat aus dem Gasgemisch (C) abgeschieden wird,
- aus dem zumindest einen Kondensat zumindest ein überwiegend Methan, Ethan und/oder Ethylen enthaltender Strom (t, d, f, h) gebildet und zumindest teilweise in die Destillationssäule (122) eingespeist wird,
- in der Destillationssäule (122) ein überwiegend Methan und Wasserstoff und ferner Ethylen enthaltendes Kopfprodukt erzeugt und zumindest teilweise als Kopfstrom (u) aus der Destillationssäule (122) abgezogen wird, und
- aus dem Kopfstrom (u) nach weiterem Abkühlen zumindest eine ethylangereicherte Flüssigkeit abgeschieden und zumindest teilweise als Rücklaufstrom (v) auf die Destillationssäule (122) aufgegeben wird,
**dadurch gekennzeichnet, dass**
für das weitere Abkühlen des Kopfstroms (u) zumindest ein Teilstrom (I) eines auf dem zweiten Temperaturniveau (T2) gasförmig verbleibenden Anteils (i) des Gasgemischs (C) verwendet wird, wobei Fluid dieses Teilstroms (I) anschließend zumindest teilweise entspannt wird.

2. Verfahren nach Anspruch 1, bei dem das Gasgemisch (C) in einer oder mehreren Stufen von dem ersten Temperaturniveau (T1) auf das zweite Temperaturniveau (T2) abgekühlt wird, wobei auf einer oder mehreren dieser Stufen Kondensate abgeschieden werden, aus denen der zumindest eine überwiegend Methan, Ethan und/oder Ethylen enthaltende Strom (t, d, f, h) gebildet wird.

3. Verfahren nach einem der Ansprüche 1 oder 2, bei dem aus dem auf dem zweiten Temperaturniveau (T2) gasförmig bleibenden Anteil (i) des Gasgemischs (C) ein weiterer Teilstrom (k) gebildet wird, der auf ein drittes Temperaturniveau (T3), das 10 K bis 50 K unterhalb des zweiten Temperaturniveaus (T2) liegt, abgekühlt und anschließend zur Bildung eines methanreichen Stroms (m) und eines wasserstoffreichen Stroms (n) verwendet wird.

4. Verfahren nach einem der vorstehenden Ansprüche, bei dem das Gasgemisch (C) auf einem Druckniveau von 10 bar bis 50 bar abgekühlt wird und/oder bei dem der Teilstrom (I) nach der Verwendung zum Abkühlen des Kopfstroms (u) zumindest teilweise auf ein Druckniveau von 5 bar bis 10 bar entspannt wird.

5. Verfahren nach einem der vorstehenden Ansprüche, bei dem der Kopfstrom (u) in mehreren Stufen abgekühlt wird, wobei auf zumindest zwei Stufen ethylenangereicherte Flüssigkeiten abgeschieden und zu dem Rücklaufstrom (v) vereinigt werden.

6. Verfahren nach einem der vorstehenden Ansprüche, bei dem ein beim Abkühlen des Kopfstroms (u) gasförmig verbleibender Anteil (x) des Kopfstroms (u) mit dem Teilstrom (I) des auf dem zweiten Temperaturniveau (T2) gasförmig bleibenden Anteils (i) des Gasgemischs (C) vereinigt und zusammen mit diesem zumindest teilweise entspannt wird.

7. Verfahren nach einem der vorstehenden Ansprüche, bei dem als Gasgemisch (C) ein Spaltgas eines Dampfspaltverfahrens oder ein aus einem derartigen Spaltgas abgeleiteter Strom verwendet wird.

8. Verfahren nach einem der vorstehenden Ansprüche, bei dem ein Mengenstrom des zum Abkühlen des Kopfstroms (u) verwendeten Teilstroms (I) in Abhängigkeit von einem Wasserstoffgehalt, einem Methangehalt und/oder einem Methan-Wasserstoff-Verhältnis des Gasgemischs (C) eingestellt wird.

9. Trennanlage (100), die zur Trennung eines Gasgemischs (C) eingerichtet ist, das überwiegend Wasserstoff, Methan, Ethan und Ethylen und wahlweise weitere Kohlenwasserstoffe enthält, mit einer Destillationssäule (122) und
- Mitteln, die dafür eingerichtet sind, das Gasgemisch (C) von einem ersten Temperaturniveau (T1) auf ein zweites Temperaturniveau (T2), das zwischen den Kondensationstemperaturen von Ethylen und Methan liegt, abzukühlen und zumindest ein Kondensat aus dem Gasgemisch (C) abzuscheiden,
- Mitteln, die dafür eingerichtet sind, aus dem zumindest einen Kondensat zumindest einen überwiegend Methan, Ethan und/oder Ethylen enthaltenden Strom (t, d, f, h) zu bilden und anschließend zumindest teilweise in die Destillationssäule (122) einzuspeisen,
- Mitteln, die dafür eingerichtet sind, in der Destillationssäule (122) ein überwiegend Methan und Wasserstoff und ferner Ethylen enthaltendes Kopfprodukt zu erzeugen und das Kopfprodukt zumindest teilweise als Kopfstrom (u) aus der Destillationssäule (122) abzuziehen, und
- Mitteln, die dafür eingerichtet sind, aus dem Kopfstrom (u) nach weiterem Abkühlen zumindest eine ethylenangereicherte Flüssigkeit abzuscheiden und die ethylenangereicherte Flüssigkeit zumindest teilweise als Rücklaufstrom (v) auf die Destillationssäule (122) aufzugeben,
**gekennzeichnet durch**
Mittel, die dafür eingerichtet sind, zum weiteren Abkühlen des Kopfstroms (u) zumindest einen Teilstrom (I) eines auf dem zweiten Temperaturniveau (T2) gasförmig bleibenden Anteils (i) des Gasgemischs (C) zu verwenden, wobei Mittel vorgesehen sind, die dafür eingerichtet sind, Fluid dieses Teilstroms (I) nach der Verwendung zum Abkühlen des Kopfstroms (u) zumindest teilweise zu entspannen.

10. Verfahren zum Umrüsten einer Trennanlage (100), die zur Trennung eines Gasgemischs (C) eingerichtet ist, das überwiegend Wasserstoff, Methan, Ethan und Ethylen und wahlweise weitere Kohlenwasserstoffe enthält, wobei die Trennanlage aufweist:
- Mittel, die dafür eingerichtet sind, das Gasgemisch (C) von einem ersten Temperaturniveau auf ein zweites Temperaturniveau (T2), das zwischen den Kondensationstemperaturen von Ethylen und Methan liegt, abzukühlen und zumindest ein Kondensat aus dem Gasgemisch (C) abzuscheiden,
- Mittel, die dafür eingerichtet sind, aus dem zumindest einen Kondensat zumindest einen überwiegend Methan, Ethan und/oder Ethylen enthaltenden Strom (t, d, f, h) zu bilden und anschließend zumindest teilweise in die Destillationssäule (122) einzuspeisen,
- Mittel, die dafür eingerichtet sind, in der Destillationssäule (122) ein überwiegend Methan und Wasserstoff und ferner Ethylen enthaltendes Kopfprodukt zu erzeugen und das Kopfprodukt zumindest teilweise als Kopfstrom (u) aus der Destillationssäule (122) abzuziehen, und
- Mittel, die dafür eingerichtet sind, aus dem Kopfstrom (u) nach weiterem Abkühlen zumindest eine ethylenangereicherte Flüssigkeit abzuscheiden und die ethylenangereicherte Flüssigkeit zumindest teilweise als Rücklaufstrom (v) auf die Destillationssäule (122) aufzugeben,
**gekennzeichnet durch**
Bereitstellen von Mitteln, die dafür eingerichtet sind, aus einem nach dem Abscheiden der Kondensate (b, d, f, h) auf dem zweiten Temperaturniveau (T2) gasförmig bleibenden Anteil (i) des Gasgemischs (C) einen weiteren Strom (I) zu bilden, den weiteren Strom (I) zum weiteren Kühlen eines nach einer ersten Abkühlung und einer ersten Phasentrennung gasförmig verbliebenen Anteils eines Kopfprodukts (u) der Destillationssäule (122) zu verwenden und danach zu entspannen.
